# FASCICULE DE BREVET EUROPEEN

(11) **EP 1 354 571 B1**
(45) Date de publication et mention de la délivrance du brevet: **06.06.2007**
(21) Numéro de dépôt: 03290973.1
(22) Date de dépôt: 18.04.2003
(51) Int. Cl.: A61F 2/38

(54) **Prothèse totale de genou**
Ganzknieprothese
Total knee prothesis

(30) Priorité: 19.04.2002 FR 0204946
(43) Date de publication de la demande: 22.10.2003
(60) Demande divisionnaire: 06076556.7
(73) Titulaire: Score PTG, 69003 Lyon (FR)
(72) Inventeur:
(74) Mandataire: Robert, Jean-Pierre

(56) Documents cités:
- FR-A- 2 796 836
- US-A- 5 011 496
- US-A- 5 935 173

## Description

La présente invention concerne une prothèse totale de genou, c'est-à-dire formée d'une pièce fémorale, une pièce tibiale et d'un insert interposé entre la pièce tibiale et la pièce fémorale.

L'articulation anatomique du genou est un joint mécanique entre l'armature osseuse de la cuisse (fémur) et l'armature osseuse de la jambe (ensemble tibia péroné) d'un membre inférieur. Ce joint mécanique est d'une cinématique complexe puisque interviennent notamment, une rotation autour d'un axe perpendiculaire au plan sagittal du membre, un déplacement de cet axe parallèlement à lui-même, une rotation-torsion autour d'un axe perpendiculaire au premier intervenant au cours de la rotation précédente, un glissement antéro-postérieur des deux pièces osseuses, chacun de ces degrés de liberté étant plus ou moins bridé ou limité par des ligaments et des tendons qui relient les armatures entre elles directement ou par l'intermédiaire des muscles d'actionnement de l'articulation.

A cette fonction de joint mécanique du genou, il s'ajoute une fonction de «poulie de renvoi à gorge » de l'effort développé par le quadriceps et appliqué à l'armature osseuse de la jambe en contournant la face antérieure du genou par l'intermédiaire du tendon rotulien. La gorge de cette «poulie» coopère au guidage et au maintien de la portion du lien entre le quadriceps et la jambe par l'intermédiaire de la rotule.

Dans cette articulation anatomique, les surfaces qui coopèrent sont cartilagineuses avec d'excellents coefficients de frottement. On y trouve également des pièces d'adaptation mobiles et déformables (par exemple les ménisques) qui ont pour fonction de rendre compliants les différents composants de l'articulation dans tous les états géométriques de l'articulation au regard de la transmission des efforts et de la répartition des pressions entre ces composants.

Lorsqu'on met en place une prothèse de genou, l'articulation ne bénéficie plus de tous les freins et attachements qui existaient naturellement entre les composants en mouvement relatifs ni d'aucune des pièces d'adaptation ou de compliance. Il est donc impossible de reconstruire une articulation de ce genou identique à l'articulation naturelle et toute prothèse n'est qu'une imitation de l'articulation anatomique.

Les prothèses modernes sont de plus en plus proches de l'articulation naturelle, non pas en termes de structure, mais en termes de fonctions assurées. C'est ainsi par exemple que le rôle de compliance des ménisques est joué par un insert qui, sans être aujourd'hui déformable, dispose d'une certaine mobilité aux lieu et place des jeux de fonctionnement entre glène et condyle qui, auparavant, étaient nécessaires à la prothèse pour s'accommoder des trajectoires relatives complexes de la cuisse et de la jambe, au détriment de la congruence des faces en contact les unes des autres donc au détriment d'une répartition harmonieuse des pressions dans l'articulation.

La présente invention ne constitue pas une rupture technologique avec l'état de la technique tel qu'il est illustré par exemple par le document US 5 609 639, US 5 935 173 et US 5 011 496. Elle concerne en revanche une multitude de perfectionnements et/ou d'améliorations de détails qui conduisent à un optimal s'approchant au plus près des fonctionnalités assurées par une articulation naturelle.

L'invention a donc pour objet une prothèse de genou comportant, comme dans l'état de la technique, une pièce fémorale, une pièce tibiale et un insert interposé entre la pièce fémorale et la pièce tibiale, la pièce fémorale comportant elle-même deux parties condyliennes réunies par une gorge intercondylienne, chaque partie condylienne ayant, dans un plan de coupe sagittal, une section limitée par un profil convexe curviligne et un profil concave polygonal, la pièce tibiale comportant un plateau et une quille sensiblement perpendiculaire au plateau et pourvue d'un alésage central ouvert sur le plateau, l'insert étant limité par une surface plane pourvue d'un plot de pivotement sur la pièce tibiale et, à l'opposé, par une surface complexe définissant deux glènes concaves séparées par un massif antéropostérieur convexe en selle de cheval.

Une prothèse de genou similaire est divulguée dans le document US 5011496. Selon une caractéristique de l'invention, dans l'état de l'articulation correspondant à l'extension du membre du patient à prothéser, la surface complexe de l'insert est totalement congruente avec la portion de surface de la pièce fémorale qui y prend appui, à l'exception de la gorge intercondylienne qui est distante du sommet du massif antéropostérieur de l'insert d'un jeu fonctionnel et chaque partie de cette surface complexe de l'insert située d'un côté du massif antéropostérieur est d'un profil sagittal médian formé par la succession de deux arcs de cercle tangents, de rayons différents, centrés sur une droite perpendiculaire à l'interligne articulaire de la prothèse, le cercle de plus petit rayon étant situé en partie postérieure de l'insert et son centre correspondant sensiblement à la projection dans le plan sagittal des épicondyles de l'articulation naturelle à prothéser.

Le jeu fonctionnel entre le sommet du massif antéropostérieur et la gorge intercondylienne a pour avantage de supprimer un hyperstatisme de la prothèse qui pourrait conduire à ce qu'il y ait un appui entre ce sommet et le fond de cette gorge formant un point de bascule de la prothèse dans le plan frontal (boitement) au niveau duquel on constaterait des concentrations importantes de contraintes en pression. Par ailleurs, la géométrie des surfaces congruentes dans le plan sagittal sous forme de deux arcs de cercle tangents, permet de respecter l'équilibre ligamentaire du genou lors de la flexion de celui-ci. La position décrite ci-dessus des deux centres des arcs de cercle de rayon différents constitue un frein à une hyper-extension de l'articulation puisque au-delà de l'extension normale, il se produirait si cela était le cas un changement brutal de centre de rotation de l'articulation conduisant à ce phénomène de freinage.

Dans un mode préféré de réalisation, la partie postérieure de surface de chaque glène de l'insert de petit rayon sagittal est une portion de sphère tandis que la partie antérieure de cette surface est une portion de tore dont le rayon des cercles méridiens est égale au rayon de la portion de sphère. Il en est bien entendu de même de la surface de chaque condyle qui coopère avec l'insert si ce n'est que les surfaces correspondantes débordent largement des glènes formées dans l'insert et notamment l'angle au centre dans le plan sagittal de la partie sphérique de chaque condyle de la partie fémorale est compris entre 90° et 110°.

En outre, chaque condyle de la partie fémorale comporte une partie terminale postérieure torique dont le profil dans un plan sagittal médian est un arc de cercle tangent au profil dans ce plan de l'extrémité postérieure de la partie sphérique, de rayon inférieur à celui de cette partie sphérique et dont le rayon d'un arc de cercle méridien est égal au rayon de la partie sphérique. Ainsi, quelle que soit l'importance surfacique du contact entre glène et condyle, l'intersection de cette surface avec un plan frontal sera toujours formée par un arc de cercle de rayon égal au rayon de la sphère susdite et d'angle au centre important pour qu'une stabilisation médio-latérale de l'articulation soit assurée quel que soit le degré de flexion de celle-ci.

La géométrie décrite ci-dessus a pour conséquence que, à partir du maximum de quantité de surface congruente de la prothèse lorsqu'elle correspond à l'extension du membre du patient, la quantité de surface congruente de chaque condyle et de chaque glène reste à peu près constante et égale à la portion sphérique postérieure de chacune des glènes de l'insert, pendant environ les soixante à soixante dix premiers degrés de flexion de l'articulation depuis son extension. Au-delà de cette flexion, la quantité de surface qui reste au contact diminue bien entendu, ce qui n'a pas de conséquence néfaste sur le fonctionnement de l'articulation car à ce degré de flexion, l'articulation est généralement beaucoup moins chargée que dans les premiers degrés de celle-ci qui correspondent au déplacement normal d'un patient dans ses activités quotidiennes (marche, gravissement ou descente d'un escalier, ...), sans pour autant que la stabilisation médio-latérale de la prothèse soit dégradée comme cela a été dit précédemment. Or une bonne stabilité médio-latérale de la prothèse à ce degré de flexion important est une qualité de celle-ci car c'est dans cette plage de flexion que l'articulation est au maximum sollicitée en torsion par ses muscles et tendons actionneurs.

Toujours de manière préférée, la gorge intercondylienne de la partie fémorale est formée de trois portions de surface torique successives dont les centres, projetés dans un plan sagittal, sont confondus avec la projection dans le même plan des centres des parties condyliennes adjacentes, le rayon de chaque cercle méridien de ces portions toriques étant unique. Il en est de même pour la surface du massif antéropostérieur de l'insert qui est formée de deux tronçons de surface torique dont le rayon de chaque cercle méridien est unique mais supérieur à celui du rayon des cercles méridiens de la gorge intercondylienne ce, afin de ménager le jeu fonctionnel susdit entre le sommet de ce massif et le fond de cette gorge. De cette manière le jeu fonctionnel est de valeur constante dans le plan sagittal médian de la prothèse, ce quel que soit son degré de flexion et diminue de part et d'autre de ce plan pour être nul dans la zone de raccordement tangentiel de chaque surface condylienne avec la gorge ou de chaque surface de glène avec le massif central.

On sait que lors de la flexion de la prothèse, il existe un mouvement antéropostérieur des condyles par rapport à l'insert. On a constaté que dans les zones d'amplitude maximale de ce mouvement et sous l'effet d'un seuil de charge relativement élevé, les forces appliquées à l'insert tendaient à l'expulser, le déboîter, de la pièce tibiale. Pour lutter contre ce risque ou ce défaut, la prothèse selon l'invention propose que le plot de pivotement de l'insert et l'alésage de la pièce tibiale pour le recevoir comportent une partie conique prolongée d'une extrémité cylindrique. La coopération de ces deux extrémités cylindriques lorsque l'insert est en appui sur la pièce tibiale interdit que l'insert bascule sous l'effet d'un effort à forte composante parallèle à l'interligne articulaire, ce qui n'est pas le cas lorsque la coopération entre le plot et l'alésage de la pièce tibiale est réalisée par un emboîtement uniquement conique.

Pour ce qui concerne la pièce fémorale, son profil concave polygonal comporte une face terminale antérieure inclinée de six degrés vers les condyles par rapport à un plan frontal perpendiculaire à l'interligne articulaire lorsque la prothèse est à zéro degré de flexion (on peut dire également que cette face est inclinée de 94° par rapport au plan de la face centrale de l'élément de prothèse, face centrale qui est parallèle au plateau tibial à zéro degré de flexion). Cette pente a été déterminée pour apporter une solution aux problèmes que pose l'adaptation d'une prothèse de genou choisie parmi une gamme de prothèses de tailles différentes alors que l'anatomie du patient s'inscrit entre deux tailles de prothèses de la gamme disponible. On rappellera à ce propos qu'il est en général préférable de mettre en place la prothèse de taille inférieure. Le choix d'une taille inférieure préserve en effet une plus grande amplitude de flexion à la prothèse. Les coupes du boîtier fémoral qui consistent à créer sur la tête de fémur cinq faces destinées à remplir le profil concave polygonal de la pièce fémorale sont repérées par rapport à des références de l'anatomie du patient selon deux ou trois techniques bien connues dans ce domaine de la chirurgie orthopédique. Parmi ces références, certains chirurgiens considèrent qu'il faut choisir les condyles postérieurs de la tête de fémur car la pièce fémorale ensuite mise en place respecte l'équilibre ligamentaire de l'articulation. Ainsi, le choix du chirurgien s'étant porté d'une part sur cette référence postérieure et d'autre part sur la mise en place d'une prothèse de taille inférieure comme dit précédemment, il se pose souvent le problème causé par l'interférence entre la partie antérieure trochléenne de la prothèse et la corticale antérieure du fémur. Avec les prothèses connues, il est souvent nécessaire d'entamer cette corticale antérieure pour que la prothèse puisse être correctement logée. L'inconvénient majeur de cette pratique réside dans le fait qu'en intervenant sur la corticale antérieure du fémur, on altère une partie saine de l'os qui est en plus celle au travers de laquelle la charge est transmise. Il n'est pas rare que cette affaiblissement soit une cause de complication à l'usage. Avec la disposition de l'invention, c'est-à-dire cette pente à six degrés de la face terminale antérieure de la partie polygonale concave de la pièce fémorale, on a toute chance de préserver l'intégrité de la corticale antérieure du fémur malgré le choix fait d'une prothèse de taille inférieure et d'une référence sur les condyles postérieurs.

De manière préférée également, la face terminale postérieure du profil concave de la pièce fémorale forme un angle d'environ deux degrés par rapport au plan frontal perpendiculaire à l'interligne articulaire.

Selon une autre caractéristique de l'invention, l'épaisseur maximale de la partie condylienne de la pièce générale, mesurée dans un plan sagittal entre chacune des trois faces du profil concave de la pièce fémorale et de son profil convexe est identique d'une part pour une même prothèse et d'autre part pour chacune des prothèses d'une gamme de prothèses selon l'invention. De manière préférée, cette épaisseur sera égale à 8 mm.

De manière avantageuse encore, la partie antérieure de la pièce fémorale comporte une gorge trochléenne en prolongation de la gorge intercondylienne dont la portion parcourue par la rotule entre zéro et environ trente degrés de flexion de la prothèse, possède un profil transversal qui correspond au profil d'un bouton rotulien en forme de calotte sphérique. Cette disposition permet de réduire les risques de luxation de la rotule par une congruence optimisée dans le sens médio-latéral de l'articulation entre le bouton rotulien qui serait rapporté derrière la rotule et le début de la gorge trochléenne de la pièce fémorale. C'est en effet au début de la flexion que les risques de luxation de la rotule sont les plus importants.

Afin d'améliorer l'ancrage de la pièce fémorale sur le fémur réséqué, les faces d'extrémité du profil concave de la partie fémorale sont pourvues de rainures dans le sens de la mise en place de cette partie sur l'os tandis que des autres faces sont pourvues d'une pluralité d'empreintes creuses comblées par l'os au moment de l'impaction.

Pour ce qui concerne à nouveau l'insert, généralement en polyéthylène, il possède une surface latérale qui présente au moins partiellement une dépouille dirigée vers l'intérieur et vers sa surface plane. Cette disposition est intéressante car elle permet d'assortir un insert donné, appairé avec la pièce fémorale de la prothèse pour des questions évidentes de congruence, avec deux voire trois pièces tibiales de tailles différentes sans que le mouvement de rotation de l'insert sur le plateau de cette pièce tibiale déborde des bords de ce dernier et cause une gêne au patient prothésé.

Enfin, il est prévu selon l'invention une gamme de prothèses dans laquelle la partie fémorale de chaque prothèse est homothétique des autres prothèses pour toutes ses dimensions à l'exception de l'épaisseur des parois qui, comme dit plus haut, reste constante quelle que soit la prothèse de la gamme. En outre, chaque insert de la gamme est homothétique des autres inserts pour ses dimensions parallèles au plan du plateau tibial. Les dimensions de ce plateau tibial appartenant à la pièce tibiale sont également homothétiques alors que la quille est de longueur unique pour toutes les pièces tibiales de la gamme de prothèse. De manière préférée, le rapport d'homothétie entre deux prothèses consécutives de la gamme est compris entre 1,047 et 1,048 et de préférence 1,0476 pour la pièce fémorale et 1,0473 pour les dimensions du plateau de la pièce tibiale et pour les dimensions de l'insert dans un plan parallèle à son plan de contact avec le plateau.

D'autres caractéristiques et avantages de la prothèse selon l'invention apparaîtront à la lumière de la description donnée ci-après de manière non limitative d'un de ses modes de réalisation.

Il sera fait référence aux dessins annexés parmi lesquels :
- la figure 1 est une vue de trois quart depuis sa face antérieure d'une prothèse selon l'invention dans une position relative des pièces qui la composent correspondant à l'extension maximale de l'articulation (0° de flexion),
- la figure 2 est une même vue de la prothèse pour une flexion de 90° de l'articulation,
- la figure 3 est une vue en coupe de la pièce fémorale et de la partie adjacente de l'insert de l'insert de la figure 1 selon le plan P1,
- la figure 4 est une vue en coupe de la pièce fémorale de la prothèse selon le plan P2 de la figure 1,

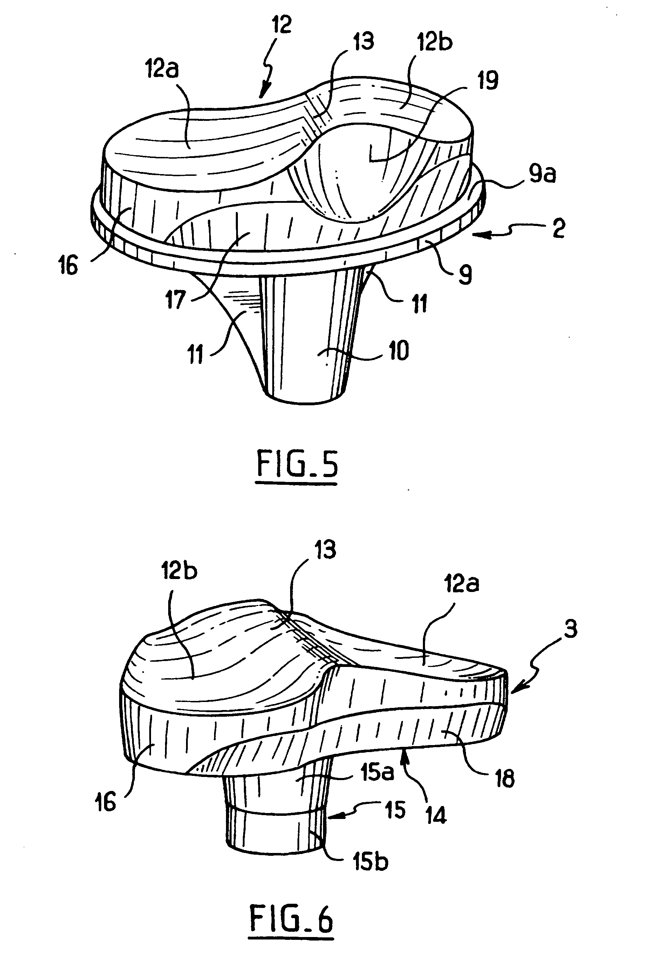

- la figure 5 est une vue extérieure de trois quarts depuis la face antérieure de la prothèse de la pièce tibiale surmontée de l'insert,
- la figure 6 est une vue de trois quarts de l'insert seul depuis sa face postérieure,
- la figure 7 est une coupe de la prothèse dans son état de la figure 1 par un plan frontal perpendiculaire à l'interligne articulaire,
- les figures 8 et 9 sont deux schémas en coupe illustrant la coopération de la gorge trochléenne de la partie fémorale de la prothèse selon l'invention avec un bouton rotulien sphérique respectivement en début de gorge trochléenne et pour une flexion de l'articulation de l'ordre de 60°.

La prothèse représentée aux dessins comporte une pièce fémorale 1, une pièce tibiale 2 et un insert 3. Les pièces fémorale et tibiale sont généralement métalliques tandis que l'insert est en matériau plastique tel que du polyéthylène. La pièce fémorale 1 est globalement divisée en deux parties : une première partie en contact glissant avec l'insert 3 qui comporte un condyle intérieur 1a et un condyle extérieur 1b réunis par une gorge intercondylienne 4. La deuxième partie de la pièce fémorale 1 n'est jamais au contact de l'insert 3 : il s'agit de la partie trochléenne 5 de la pièce fémorale comportant, en prolongement des condyles 1a et 1b des berges intérieure 5a et extérieure 5b séparées par une gorge trochléenne 6. Cette pièce fémorale, en forme de coquille, possède une surface extérieure complexe lisse, globalement convexe et est limitée à l'intérieur par une surface concave formée de cinq faces successives repérées 7a à 7e depuis la partie postérieure jusqu'à la partie antérieure de la pièce fémorale, délimitant un volume prismatique. La face centrale 7c est pourvue de pions d'ancrage 8 de la prothèse dans la tête fémorale réséquée.

La pièce tibiale 2 est de manière classique composée d'un plateau supérieur 9 surfacé (poli-miroir) sur sa face supérieure 9a, et comportant une quille 10 d'implantation dans le tibia avec des ailerons 11, la quille 10 étant creuse pour d'une part être fixée au moyen de cônes morses à une tige implantée dans le tibia et d'autre part, recevoir un plot de pivotement de l'insert 3.

L'insert 3 quant à lui est une pièce en polyéthylène qui présente une surface supérieure 12 complexe divisée en deux glènes 12a, 12b, séparées par un massif antéropostérieur 13. A l'opposé de cette surface supérieure 12, l'insert présente une surface inférieure plane 14 destinée à reposer sur la surface supérieure 9a de la pièce tibiale et dans la partie médiane de laquelle, au droit du massif 13, est implanté un pivot 15 conique dans sa partie 15a proche de la surface 14 et cylindrique à son extrémité 15b. L'alésage prévu pour le recevoir dans la quille 10 de la pièce tibiale 2 est de même forme.

L'insert 3 délimité latéralement par une surface 16 dont le contour est en forme de haricot, comme le contour du plateau tibial et ce de manière connue, cette surface 16 possédant une dépouille antérieure 17 inclinée vers le centre en direction de la surface 14 et une dépouille postérieure 18 également inclinée vers le centre et vers la surface 14. On notera également la présence d'un embrèvement 19 à la jonction du massif antéropostérieur 13 et de la partie antérieure de la surface latérale 16 de l'insert, embrèvement qui sert à dégager un espace de logement du tendon rotulien lorsque l'articulation est en flexion maximale alors qu'il s'est produit un mouvement antéropostérieur de la pièce fémorale par rapport à la pièce tibiale dans cet état.

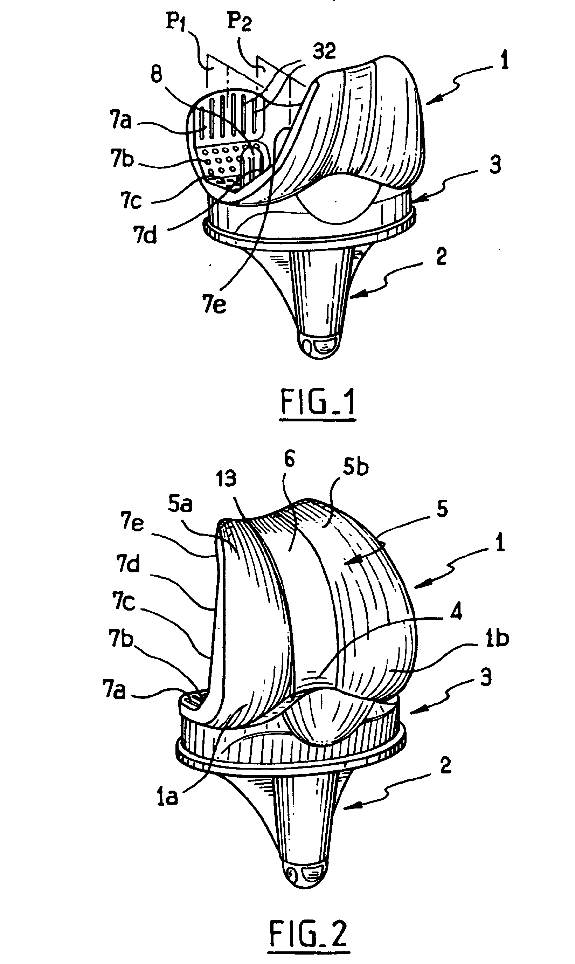

A la figure 1 on a mentionné deux plans, à savoir un plan sagittal P1 passant par la partie médiane du condyle interne 1a de la pièce fémorale et un plan sagittal P2 passant par la partie médiane de la gorge intercondylienne. A cette figure, la prothèse est représentée dans l'état d'extension maximal (0° degré de flexion) de l'articulation, c'est-à-dire que la face centrale 7c du profil concave de la pièce fémorale est sensiblement parallèle à la surface supérieure 9a du plateau tibial et donc parallèle à ce qu'il est connu d'appelé l'interligne articulaire.

A la figure 2, la pièce fémorale a basculé de 90° par rapport à la pièce tibiale, ce qui correspond à une position de la face 7c sensiblement perpendiculaire à la face 9a de la pièce tibiale.

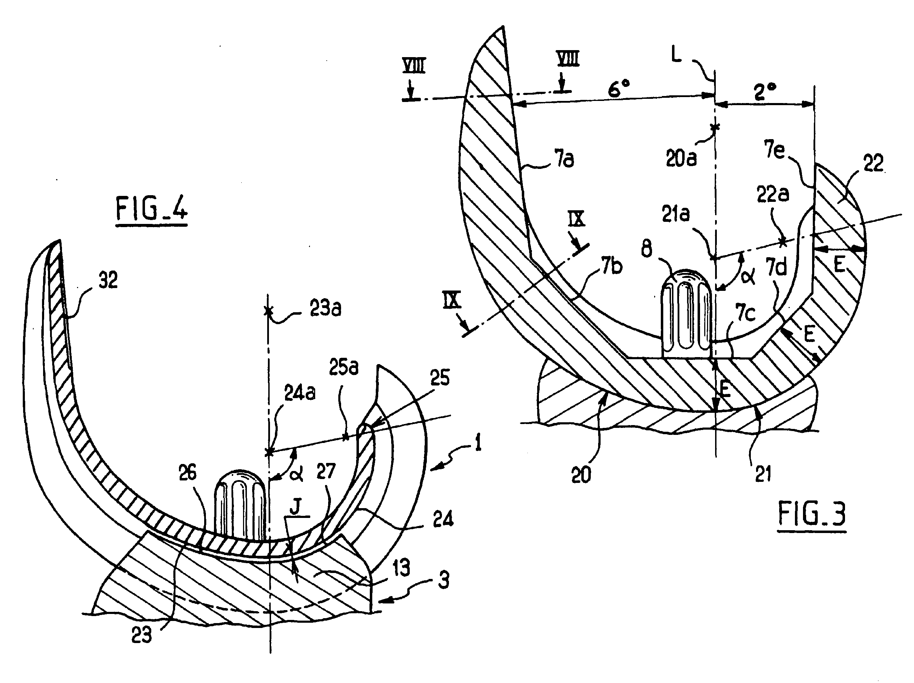

Dans sa position de la figure 1, la prothèse est telle que la portion de surface condylienne en appui sur l'insert 3 et la surface supérieure de cet insert 3 sont en congruence maximale, c'est-à-dire qu'elles se correspondent point à point à l'exception du sommet du massif antéropostérieur 13 et du fond de la gorge intercondylienne où il existe un jeu fonctionnel J. Cette congruence et ce jeu sont illustrés par les figures 3, 4 et 7.

La figure 3 est une coupe de la partie fémorale de la prothèse et de la partie supérieure de l'implant par le plan P1 lorsque la prothèse est dans sa position de la figure 1. Le profil dans ce plan du condyle intérieur 1a, qui est le même que le profil du condyle extérieur 1b dans un plan parallèle au plan P1, ainsi que le profil de la glène intérieure 12a de l'insert, est formé par la succession de deux arcs de cercle 20 et 21 dont les centres 20a et 21a sont situés sur une ligne L perpendiculaire à la face 7c centrale du profil interne de la prothèse, donc perpendiculaire à l'interligne articulaire. Ces arcs de cercle sont tangents l'un à l'autre et l'arc de cercle postérieur 21 est de rayon plus petit que l'arc de cercle antérieur 20. Le centre 21a de l'arc de cercle postérieur 21 et situé sur la ligne frontale qui rejoint les épicondyles de l'articulation naturelle du patient à prothéser. Sur les condyles 1a et 1b de la partie fémorale, cet arc de cercle possède un angle au centre ∝ compris entre 90° et 110° et de préférence égal à 98°. Au-delà de cet arc de cercle 21, la partie postérieure de chaque condyle se termine par une surface dont le profil dans le plan P1 est également constitué par un arc de cercle 22 tangent à l'arc de cercle 21 et dont le rayon est inférieur à celui de l'arc de cercle 21. La surface antérieure de la partie trochléenne de la pièce fémorale est raccordée à l'arc de cercle 20 et possède une forme classique, dictée par l'anatomie de la jambe et le guidage de la rotule.

A la figure 4, c'est-à-dire dans le plan de coupe P2 de l'articulation, le fond de la gorge intercondylienne présente le même profil d'arcs de cercle successifs que précédemment décrits avec bien entendu des rayons plus petits. Ainsi, on retrouve au fond de la gorge intercondylienne, un premier arc de cercle 23 de centre 23a, tangent à un second arc de cercle 24 de centre 24a et raccordé à un troisième arc de cercle 25 de centre 25a. Les centres 23a, 24a et 25a ont une projection identique à celle des centres 20a, 21a et 22a dans un plan sagittal quelconque de l'articulation. De la même manière, le sommet du massif 13 de l'insert 3 a un profil dans le plan P2 formé de deux arcs de cercle successifs 26, 27 raccordés de manière tangentielle et de centre les centres 23a, 24a. Le rayon des arcs de cercle 26 et 27 est légèrement supérieur à celui des arcs de cercle 23 et 24 de manière à laisser subsister le jeu fonctionnel J précédemment évoqué.

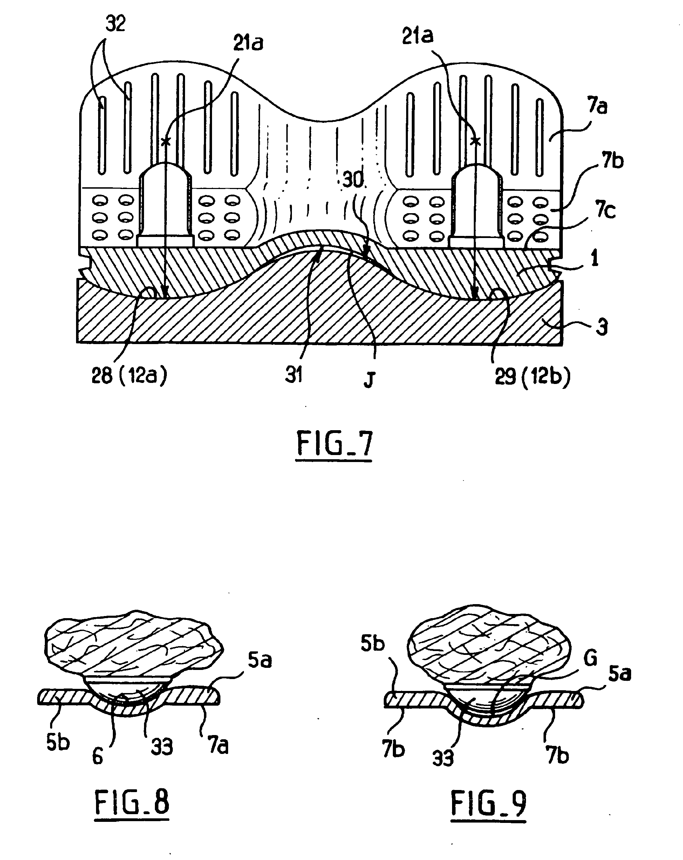

La figure 7 représente une coupe partielle frontale de la prothèse, limitée à la partie fémorale et à l'insert, pour illustrer la congruence médiolatérale de ces deux éléments. Cette coupe frontale passe par la ligne L des centres 20a et 21a susdits. Le profil des condyles 1a et 1b et des glènes 12a, 12b dans ce plan est un arc de cercle 28, 29 ayant pour centre le centre 21a susdit. Cela signifie que le rayon de ces arcs de cercle est identique au rayon de l'arc de cercle 21, ce qui implique que la surface postérieure de chaque glène est une portion de surface sphérique et que la surface postérieure de chaque condyle embrassé par l'angle ∝ est également une portion de surface sphérique.

On notera également sur la figure 7 la représentation du jeu J dont la forme en croissant est définie par la différence entre les rayons des arc de cercle 30 et 31 forment respectivement le sommet du massif et le fond de la gorge intercondylienne, le rayon du premier étant supérieur au rayon de l'autre.

Les profils représentés à la figure 7 sont constants quels que soient les plans de coupe de la partie condylienne de la pièce fémorale et de l'insert 3, plans de coupe radiaux passant par le centre 20a pour la partie antérieure de la prothèse (profil sagittal 20), par le centre 21a pour la partie postérieure de la prothèse (profil sagittal 21) et passant par le centre 22a pour l'extrémité postérieure de la partie condylienne de la pièce fémorale. On comprend que, même si au cours de la flexion les surfaces en congruence diminuent, la congruence médiolatérale de l'articulation reste importante.

Une autre des caractéristiques de la prothèse selon l'invention est que, en référence à la figure 3, l'épaisseur maximale E de chaque condyle, en regard des faces 7c, 7d, 7e de la partie fémorale, est identique pour chacune de ces trois parties. Elle sera de préférence déterminée égale à 8mm quelle que soit la taille de prothèse mise en place. On notera également en regard de cette figure 3, l'inclinaison de la face antérieure 7a de la partie interne de la pièce fémorale égale à 6° sur un plan frontal perpendiculaire à la face 7c pour résoudre le problème de l'interférence avec 1 corticale antérieure du fémur. La face postérieure 7e est quant à elle inclinée de 2° par rapport à ce plan. Les faces 7a et 7e sont pourvues de stries ou rainures parallèles et orientées dans le sens de la mise en place de la prothèse sur le fémur, ces rainures sont référencées 32 sur les figures 1, 4 et 7. Les autres faces 7b, 7c et 7d sont quant à elles pourvues de petites empreintes en cuvettes ménagées en creux ayant pour effet d'augmenter la surface en contact avec l'os.

En se reportant aux figures 8 et 9, correspondant aux coupes VIII-VIII et IX-IX indiquées à la figure 3, on retrouve la partie trochléenne 5 de la pièce fémorale 1 avec la gorge trochléenne 6 qui, dans le cas de la figure 8, est d'un profil identique à celui d'un bouton rotulien 32 sphérique, de sorte que la congruence médiolatérale de la gorge 6 avec le bouton 32 soit maximale dans la partie terminale de la gorge trochléenne 6. La coupe de la figure 9 correspond à la zone trochléenne qui est la plus voisine de celle de la gorge intercondylienne. Dans cette position, le bouton rotulien 32 coopère avec la gorge 6 de manière moins congruente, ce qui ne présente pas de risque à l'égard d'une luxation de la rotule car dans cette position la rotule a été suffisamment guidée et engagée dans la gorge pour ne plus s'en échapper et ce qui présente l'avantage d'autoriser une certaine rotation de la rotule sur elle-même pour prendre en compte les différents mouvements parasites de l'articulation lors de la flexion.

La gamme de prothèses construites conformément à ce qui est décrit ci-dessus comporte de préférence sept tailles. Les pièces fémorales varient d'une taille à l'autre dans un rapport d'homothétie égal à 1,00476 à l'exception des épaisseurs notamment celles E qui restent identiques pour chaque taille donc égales à 8 mm. La taille No 4 de cette prothèse sera telle que le rayon des arcs de cercle 21, 28 et 29 sera égal à 24 mm. Pour ce qui concerne la pièce tibiale, seul le plateau varie de manière homothétique d'une pièce à l'autre dans la gamme des sept éléments prévus. Le rapport d'homothétie a été déterminé comme étant optimal à la valeur de 1,0473.

Enfin, pour ce qui concerne l'insert, seules les dimensions parallèles au plan de sa face 14 varient dans les mêmes proportions que celles du plateau tibial dans la gamme prévue. Grâce aux dépouilles 17 et 18, il est cependant possible d'assortir une pièce tibiale d'une taille déterminée avec un insert et une pièce fémorale d'une taille inférieure ou supérieure sans que l'insert déborde de manière préjudiciable du plateau 9 de la pièce tibiale.

## Revendications

1. Prothèse de genou comportant une pièce fémorale (1), une pièce tibiale (2) et un insert (3) interposé entre la pièce fémorale et la pièce tibiale, la pièce fémorale comportant deux parties condyliennes (1a, 1b) réunies par une gorge intercondylienne (4) ayant, dans un plan de coupe sagittal, une section limitée par un profil convexe curviligne et un profil concave polygonal, la pièce tibiale comportant un plateau (9) et une quille (10) sensiblement perpendiculaire au plateau et pourvue d'un alésage central ouvert sur le plateau, l'insert étant limité par une surface plane (14) pourvue d'un plot (15) de pivotement sur la pièce tibiale (2) et, à l'opposé, par une surface complexe (12) définissant deux glènes (12a, 12b) concaves, séparées par un massif antéropostérieur (13) convexe en selle de cheval, **caractérisée en ce que**, dans l'état de l'articulation correspondant à l'extension de la jambe du patient prothésé, la surface complexe (12) de l'insert est totalement congruente avec la portion de surface de la pièce fémorale qui y prend appui, à l'exception du fond de la gorge intercondylienne (4) qui est distante du sommet du massif antéropostérieur (13) de l'insert d'un jeu fonctionnel (J) et **en ce que** chaque partie de cette surface complexe (12) de l'insert (2), située d'un côté du massif antéropostérieur (13) possède un profil sagittal médian formé par la succession de deux arcs de cercle (20, 21) tangents, de rayons différents, centrés sur une droite (L) perpendiculaire à l'interligne articulaire de la prothèse, le cercle (21) de plus petit rayon étant situé en partie postérieure de l'insert (3) et son centre (21a) correspondant sensiblement à la trace dans le plan sagittal de la ligne reliant les épicondyles de l'articulation naturelle à prothéser.

2. Prothèse selon la revendication 1, **caractérisée en ce que** la partie postérieure de surface de chaque glène (12a, 12b) de l'insert de petit rayon sagittal (21) est une portion de sphère tandis que la partie antérieure de cette surface est une portion de tore dont le rayon des cercles méridiens est égal au rayon de la portion de sphère.

3. Prothèse selon la revendication 1 ou la revendication 2, **caractérisée en ce que** l'angle α au centre dans un plan sagittal de la partie sphérique de chaque condyle (1a, 1b) de la partie fémorale 1 est compris entre 90°et 110°.

4. Prothèse selon la revendication 3, **caractérisée en ce que** chaque condyle de la partie fémorale (1) comporte une partie terminale postérieure torique dont le profil dans un plan sagittal médian est un arc de cercle (22) tangent au profil dans ce plan de l'extrémité postérieure de la partie sphérique, de rayon inférieur à celui de cette partie sphérique et dont le rayon d'un arc de cercle méridien est égal au rayon de la partie sphérique.

5. Prothèse selon la revendication 4, **caractérisée en ce que** la gorge (4) intercondylienne de la partie fémorale est formée de trois portions de surface toriques successives dont les centres (23a, 24a et 25a) projetés dans le plan sagittal sont confondus avec les projections (20a, 21a, 22a) dans le même plan des centres des parties condyliennes adjacentes, le rayon de chaque arc de cercle méridien (31) de ces portions toriques étant unique.

6. Prothèse selon la revendication 5, **caractérisée en ce que** le sommet du massif antéropostérieur est formé, comme la gorge intercondylienne, de trois portions de surface torique successives de même centre que ceux de la gorge intercondylienne, le rayon de chaque arc de cercle (30) méridien de ces portions toriques étant unique et supérieur au rayon correspondant de la gorge intercondylienne.

7. Prothèse selon l'une quelconque des revendications précédentes, **caractérisée en ce que** le plot (15) de pivotement de l'insert (3) et l'alésage de la pièce tibiale pour le recevoir comporte une partie conique (15a) prolongée d'une extrémité cylindrique (15b).

8. Prothèse selon l'une quelconque des revendications précédentes, **caractérisée en ce que** le profil concave polygonal de la pièce fémorale (1) comporte une face terminale (7a) antérieure inclinée de 6° vers les condyles par rapport à un plan frontal perpendiculaire à l'interligne articulaire.

9. Prothèse selon la revendication 8, **caractérisée en ce que** la face terminale postérieure (7e) du profil concave de la pièce fémorale (1) forme un angle de 2° par rapport au plan frontal susdit.

10. Prothèse selon l'une quelconque des revendications précédentes, **caractérisée en ce que** l'épaisseur maximale (E) de la paroi de la pièce fémorale (1) au niveau de sa partie condylienne, mesurée perpendiculairement à chaque face (7c, 7d, 7e) du profil concave, est constante.

11. Prothèse selon l'une quelconque des revendications précédentes, **caractérisée en ce que** la partie antérieure (5) de la pièce fémorale comporte une gorge trochléenne (6) en prolongation de la gorge intercondylienne (4) dont la portion parcourue par la rotule entre 0° et environ 30° de flexion de la prothèse, possède un profil transversal qui correspond au profil d'un bouton rotulien (32) en forme de calotte sphérique ;

12. Prothèse selon l'une quelconque des revendications précédentes, **caractérisée en ce que** les faces d'extrémité (7a, 7e) du profil concave de la partie fémorale sont pourvues de rainures (32) orientées dans le sens de la mise en place de cette partie sur l'os.

13. Prothèse selon la revendication 12, **caractérisée en ce que** les autres faces (7b, 7c, 7d) du profil concave de la pièce fémorale (1) sont pourvues d'une pluralité d'empreintes creuses.

14. Prothèse selon l'une quelconque des revendications précédentes, **caractérisée en ce que** la surface latérale (16) de l'insert est au moins partiellement en dépouille (17, 18) vers l'intérieur en direction de sa surface plane (14).

15. Gamme de prothèses selon l'une quelconque des revendications précédente, **caractérisée en ce que** la partie fémorale de chaque prothèse de la gamme est homothétique des autres prothèses pour toutes ses dimensions à l'exception de l'épaisseur des parois qui reste constante quelle que soit la prothèse de la gamme.

16. Gamme de prothèses selon la revendication 15, **caractérisée en ce que** chaque insert de la gamme est homothétique des autres inserts pour ses dimensions parallèles à sa surface inférieure plane (14) d'appui sur le plateau tibial (9), les dimensions de ces derniers dans ce plan étant également homothétiques.

17. Gamme de prothèses selon les revendications 15, 16, **caractérisée en ce que** le rapport d'homothétie entre deux prothèses consécutives de la gamme est compris entre 1,047 et 1,048 et de préférence 1,0476 pour la pièce fémorale (1) et 1,0473 pour la pièce tibiale (2) et l'insert (3).

## Claims

1. A knee prosthesis comprising a femur piece (1), a tibia piece (2), and an insert (3) interposed between the femur piece and the tibia piece, the femur piece including two condyl portions (1a, 1b) united by an intercondyl groove (4) and having, in a sagittal section plane, a section that is limited by a curvilinear convex profile and a polygonal concave profile, the tibia piece comprising a platform (9) and a shank (10) substantially perpendicular to the platform and provided with a central bore that opens into the platform, the insert being limited by a plane surface (14) provided with a stud (15) for pivoting on the tibia piece (2) and, opposite therefrom, by a complex surface (12) defining two concave sockets (12a, 12b) separated by a saddle-shaped convex anteroposterior ridge (13), the prosthesis being **characterized in that** in the state of the joint corresponding to extension of the leg of the patient fitted with the prosthesis, the complex surface (12) of the insert is completely congruent with the surface portion of the femur piece that bears thereagainst, with the exception of the bottom of the intercondyl groove (4) which is spaced apart from the top of the anteroposterior ridge (13) of the insert by functional clearance (J), and **in that** each portion of said complex surface (12) of the insert (2) situated on one side of the anteroposterior ridge (13) possesses a median sagittal profile shaped by a succession of two tangential circular arcs (20, 21) of different radii centered on a straight line (L) perpendicular to the joint space of the prosthesis, the circle (21) of smaller radius being situated in the posterior portion of the insert (3) and its center (21a) corresponding substantially to the trace in the sagittal plane of the line interconnecting the epicondyls of the natural joint to which the prosthesis is to be fitted.

2. A prosthesis according to claim 1, **characterized in that** the posterior portion of the surface of each socket (12a, 12b) of the insert of small sagittal radius (21) is a portion of a sphere, while the anterior portion of said surface is a portion of a torus with meridian circles having a radius equal to the radius of the portion of a sphere.

3. A prosthesis according to claim 1 or claim 2, **characterized in that** the angle α at the center in a sagittal plane of the spherical portion of each condyl (1a, 1b) of the femur piece 1 lies in the range 90° to 110°.

4. A prosthesis according to claim 3, **characterized in that** each condyl of the femur piece (1) has a toroidal posterior terminal portion of profile in a median sagittal plane that is a circular arc (22) tangential to the profile in said plane of the posterior end of the spherical portion, of radius less than that of said spherical portion, and for which the radius of a meridian circular arc is equal to the radius of the spherical portion.

5. A prosthesis according to claim 4, **characterized in that** the intercondyl groove (4) of the femur piece is made up of three successive toroidal surface portions having centers (23a, 24a, and 25a) projected into the sagittal plane that coincide with the projections (20a, 21a, 22a) in the same plane of the centers of the adjacent condyl portions, the radius of each meridian circular arc (31) of these toroidal portions being the same.

6. A prosthesis according to claim 5, **characterized in that**, like the intercondyl groove, the top of the anteroposterior ridge is made up of three successive toroidal surface portions having the same centers as the portions of the intercondyl groove, the radius of each meridian circular arc (30) of these toroidal portions being the same, and greater than the corresponding radius of the intercondyl groove.

7. A prosthesis according to any preceding claim, **characterized in that** the pivot stud (15) of the insert (3) and the bore of the tibia piece for receiving it comprise a conical portion (15a) extended by a cylindrical end (15b).

8. A prosthesis according to any preceding claim, **characterized in that** the polygonal concave profile of the femur piece (1) includes an anterior terminal face (7a) inclined at 6° towards the condyls relative to a frontal plane perpendicular to the joint space.

9. A prosthesis according to claim 8, **characterized in that** the posterior terminal face (7e) of the concave profile of the femur piece (1) forms an angle of 2° relative to the above-mentioned frontal plane.

10. A prosthesis according to any preceding claim, **characterized in that** the maximum thickness (E) of the wall of the femur piece (1) level with its condyl portion, measured perpendicularly to each face (7c, 7d, 7e) of the concave profile, is constant.

11. A prosthesis according to any preceding claim, **characterized in that** the anterior portion (5) of the femur piece includes a trochlear groove (6) extending the intercondyl groove (4) in which the portion followed by the patella between 0° and about 30° of flexing of the prosthesis possesses a transverse profile that corresponds to the profile of a patellar button (32) in the form of a spherical cap.

12. A prosthesis according to any preceding claim, **characterized in that** the end faces (7a, 7e) of the concave profile of the femur piece are provided with grooves (32) oriented in the direction this piece is put into place on the bone.

13. A prosthesis according to claim 12, **characterized in that** the other faces (7b, 7c, 7d) of the concave profile of the femur piece (1) are provided with a plurality of hollow indentations.

14. A prosthesis according to any preceding claim, **characterized in that** at least part of the side surface (16) of the insert tapers (17, 18) inwards towards its plane surface (14).

15. A set of prostheses according to any preceding claim, **characterized in that** the femur piece of each prosthesis in the set is geometrically similar to all of the other prostheses concerning all of its dimensions, with the exception of its wall thicknesses that remain constant for all of the prostheses in the set.

16. A set of prostheses according to claim 15, **characterized in that** each insert of the set is geometrically similar to the other insert for all of its dimensions parallel to its plane bottom surface (14) for bearing against the tibia platform (9), with the dimensions thereof in said plane likewise being geometrically similar.

17. A set of prostheses according to claim 15 or claim 16, **characterized in that** the scaling ratio between two consecutive prostheses in the set lies in the range 1.047 to 1.048, and is preferably 1.0476 for the femur piece (1) and 1.0473 for the tibia piece (2) and the insert (3).

## Patentansprüche

1. Knieprothese, umfassend einen femoralen Teil (1), einen tibialen Teil (2) und einen Einsatz (3), der zwischen dem femoralen Teil und dem tibialen Teil angeordnet ist, wobei der femorale Teil zwei kondyläre Abschnitte (1a, 1b) umfasst, die über eine interkondyläre Kehle (4) verbunden sind, die in einer sagitallen Schnittebene einen Querschnitt hat, der von einem konvexen gekrümmten Profil und einem konkaven polygonalen Profil begrenzt ist, wobei der tibiale Teil eine Platte (9) und einen Schaft (10) umfasst, der im Wesentlichen senkrecht zur Platte und mit einer zur Platte hin offenen zentralen Bohrung versehen ist, wobei der Einsatz von einer ebenen Fläche (14) begrenzt ist, die mit einem Schwenkzapfen (15) für ein Drehen an dem tibialen Teil (2) versehen ist, sowie auf der anderen Seite von einer komplexen Fläche (12), die zwei konkave Gelenkpfannen (12a, 12b) definiert, die durch eine anteroposteriore konvexe sattelförmige Erhebung (13) getrennt sind, **dadurch gekennzeichnet, dass** in dem Gelenkverbindungszustand, der dem Strecken des Beines des eine Prothese tragenden Patienten entspricht, die komplexe Fläche (12) des Einsatzes vollkommen kongruent mit dem Oberflächenabschnitt des femoralen Teils ist, das dort zur Anlage kommt, mit Ausnahme des Bodens der interkondylären Kehle (4), die zum Scheitel der anteroposterioren Erhebung (13) des Einsatzes um ein funktionales Spiel (J) beabstandet ist, und dass jeder Abschnitt dieser komplexen Fläche (12) des Einsatzes (2), der sich auf einer Seite der anteroposterioren Erhebung befindet, ein Mediansagittalprofil hat, das durch das Aufeinanderfolgen von zwei unterschiedliche Radien aufweisenden, sich berührenden Kreisbögen (20, 21) gebildet ist, deren Mittelpunkte auf einer Geraden (L) liegen, die senkrecht zum Gelenkspalt der Prothese ist, wobei sich der Kreisbogen (21) mit dem kleineren Radius auf dem posterioren Abschnitt des Einsatzes (3) befindet und sein Mittelpunkt (21a) in der Sagittalebene im Wesentlichen der Spur der Linie entspricht, die die Epikondylen des natürlichen, mit einer Prothese zu versehenden Gelenks verbindet.

2. Prothese nach Anspruch 1, **dadurch gekennzeichnet, dass** der posteriore Flächenabschnitt jeder Gelenkpfanne (12a, 12b) des Einsatzes mit kleinem Sagittalradius (21) ein Kugelabschnitt ist, während der anteriore Abschnitt dieser Fläche ein Torusabschnitt ist, bei dem der Radius der meridionalen Kreise gleich dem Radius des Kugelabschnittes ist.

3. Prothese nach Anspruch 1 oder Anspruch 2, **dadurch gekennzeichnet, dass** der Winkel α im Mittelpunkt in einer Sagittalebene des Kugelabschnittes jedes Kondylus (1a, 1b) des femoralen Teils (1) zwischen 90° und 110° beträgt.

4. Prothese nach Anspruch 3, **dadurch gekennzeichnet, dass** jeder Kondylus des femoralen Abschnittes (1) einen torischen posterioren Endabschnitt umfasst, dessen Profil in einer Mediansagittalebene ein Kreisbogen (22) ist, der tangential zum Profil des posterioren Endes des Kugelabschnittes in dieser Ebene ist, mit einem Radius, der kleiner ist als der dieses Kugelabschnittes, und dessen Radius eines meridionalen Kreisbogens gleich dem Radius des Kugelabschnittes ist.

5. Prothese nach Anspruch 4, **dadurch gekennzeichnet, dass** die interkondyläre Kehle (4) des femoralen Abschnittes aus drei aufeinanderfolgenden torischen Oberflächenabschnitten gebildet ist, deren Mittelpunkte (23a, 24a und 25a), projiziert auf die Sagittalebene, mit den Projektionen (20a, 21a, 22a) der Mittelpunkte der angrenzenden kondylären Abschnitte in der gleichen Ebene zusammenfallen, wobei der Radius jedes meridionalen Kreisbogens (31) dieser torischen Abschnitte einzigartig ist.

6. Prothese nach Anspruch 5, **dadurch gekennzeichnet, dass** der Scheitel der anteroposterioren Erhebung wie die interkondyläre Kehle aus drei aufeinanderfolgenden torischen Oberflächenabschnitten mit dem gleichen Mittelpunkt wie die der interkondylären Kehle gebildet ist, wobei der Radius jedes meridionalen Kreisbogens (30) dieser torischen Abschnitte einzigartig und größer als der entsprechende Radius der interkondylären Kehle ist.

7. Prothese nach einem der vorhergehenden Ansprüche, **dadurch gekennzeichnet, dass** der Zapfen (15) für das Drehen des Einsatzes (3) und die Bohrung des tibialen Teils für seine Aufnahme einen konischen Abschnitt (15a) umfassen, der durch ein zylindrisches Ende (15b) verlängert ist.

8. Prothese nach einem der vorhergehenden Ansprüche, **dadurch gekennzeichnet, dass** das polygonale konkave Profil des femoralen Teils (1) eine anteriore Endfläche (7a) umfasst, die um 6° zu den Kondylen hin relativ zu einer Frontalebene geneigt ist, die senkrecht zum Gelenkspalt ist.

9. Prothese nach Anspruch 8, **dadurch gekennzeichnet, dass** die posteriore Endfläche (7e) des konkaven Profils des femoralen Teils (1) einen Winkel von 2° relativ zur oben genannten Frontalebene bildet.

10. Prothese nach einem der vorhergehenden Ansprüche, **dadurch gekennzeichnet, dass** die maximale Stärke (E) der Wand des femoralen Teils (1) im Bereich ihres kondylären Abschnittes, gemessen senkrecht zu jeder Fläche (7c, 7d, 7e) des konkaven Profils, konstant ist.

11. Prothese nach einem der vorhergehenden Ansprüche, **dadurch gekennzeichnet, dass** der anteriore Abschnitt (5) des femoralen Teils in der Verlängerung der interkondylären Kehle (4) eine trochleare Kehle (6) umfasst, deren von der Kniescheibe bei einer Biegung der Prothese zwischen 0° und ungefähr 30° durchlaufener Abschnitt ein transversales Profil hat, das dem Profil eines Patellaknopfes (33) in Form einer Kugelkappe entspricht.

12. Prothese nach einem der vorhergehenden Ansprüche, **dadurch gekennzeichnet, dass** die Endflächen (7a, 7e) des konkaven Profils des femoralen Abschnittes mit Nuten (32) versehen sind, die in Richtung des Platzierens dieses Abschnittes auf dem Knochen ausgerichtet sind.

13. Prothese nach Anspruch 12, **dadurch gekennzeichnet, dass** die anderen Flächen (7b, 7c, 7d) des konkaven Profils des femoralen Teils (1) mit einer Vielzahl von hohlen Vertiefungen versehen sind.

14. Prothese nach einem der vorhergehenden Ansprüche, **dadurch gekennzeichnet, dass** die seitliche Fläche (16) des Einsatzes mindestens teilweise nach Innen in Richtung ihrer planen Oberfläche (14) abgeschrägt ist.

15. Prothesensortiment nach einem der vorhergehenden Ansprüche, **dadurch gekennzeichnet, dass** der femorale Abschnitt jeder Prothese aus dem Sortiment homothetisch zu den anderen Prothesen ist, hinsichtlich all seiner Abmessungen mit Ausnahme der Dicke der Wände, die unabhängig von der aus dem Sortiment ausgewählten Prothese konstant ist.

16. Prothesensortiment nach Anspruch 15, **dadurch gekennzeichnet, dass** jeder Einsatz aus dem Sortiment homothetisch zu den anderen Einsätzen ist, hinsichtlich seiner Abmessungen, die parallel zu seiner ebenen unteren Fläche (14) zur Anlage an der tibialen Platte (9) sind, wobei die Abmessungen dieser letztgenannten in dieser Ebene ebenfalls homothetisch sind.

17. Prothesensortiment nach den Ansprüchen 15, 16, **dadurch gekennzeichnet, dass** das Verhältnis der Homothetie zwischen zwei aufeinanderfolgenden Prothesen aus dem Sortiment zwischen 1,047 und 1,048 und vorzugsweise 1,0476 für den femoralen Teil (1) und 1,0473 für den tibialen Teil (2) und den Einsatz (3) beträgt.
